# EUROPEAN PATENT SPECIFICATION

(11) **EP 1 294 360 B1**
(45) Date of publication and mention of the grant of the patent: **12.10.2005**
(21) Application number: 01943685.6
(22) Date of filing: 29.06.2001
(51) Int. Cl.: A61K 9/16

(54) **PROCESS FOR THE PREPARATION OF CRYSTALLINE PARTICLES FOR INHALATION**
VERFAHREN ZUR HERSTELLUNG KRISTALLINEr PARTIKEL ZUR INHALATION
PROCEDE DE PREPARATION DES PARTICULES CRISTALLINES POUR INHALATION

(30) Priority: 29.06.2000 GB 0016040
(43) Date of publication of application: 26.03.2003
(73) Proprietor: GLAXO GROUP LIMITED, Middlesex UB6 0NN (GB)
(72) Inventor: LANCASTER, Robert William, GlaxoSmithKline, Stevenage, Hertfordshire SG1 2NY (GB); SINGH, Hardev, GlaxoSmithKline, Dartford, Kent DA1 5AH (GB); THEOPHILUS, Andrew Lewis, GlaxoSmithKline, Stevenage, Hertfordshire SG1 2NY (GB)
(74) Representative: Giddings, Peter John
(86) International application number: PCT/GB2001/002923
(87) International publication number: WO 2002/000199

(56) References cited:
- WO-A-00/38811
- WO-A-00/44468
- WO-A-96/32095

## Description

This invention relates to a novel process for preparing crystalline particles, particularly particles of defined particle size distribution, especially particles of therapeutically useful or carrier substances of a size suitable for inhalation therapy.

Industrial processes for production of many products, particularly pharmaceutical products, require the preparation of pure substances of a defined particle size distribution. Pure substances are frequently prepared by precipitation from solutions of lesser purity. When precipitation takes place relatively slowly (e.g. over a matter of hours), crystals are grown which are frequently of a non-uniform shape and relatively large size.

In the field of inhalation therapy, therapeutic molecules are generally desired of a particle size "suitable for inhalation", which is a term generally taken to indicate an aerodynamic diameter between 1 and 10 µm, especially 1 and 5 µm, particularly 1 and 3 µm. Carrier molecules (such as lactose) for inhaled therapeutic preparations are typically desired of a significantly larger aerodynamic diameter so that they do not penetrate into the upper respiratory tract to the same degree as the active ingredient and an aerodynamic diameter of 100 to 150 µm is generally considered suitable. However this is a generalisation and for some purposes it may well be preferred to use a lower particle size for the carrier, even one comparable to that of the therapeutic substance.

Outside of the inhaled area, modification of the habit and size of crystals is a valuable tool in adjusting and optimising pharmaceutical and biological properties such as flow characteristics, dissolution rate and bioavailability.

Particles of the desired particle size for inhalation therapy are conventionally prepared by milling or micronisation. These processes, depending on the precise conditions adopted, are capable of generating particle distributions which include fractions having particles with the appropriate size. Milling is suitable for preparing particles of the larger size indicated above and micronisation of the smaller size indicated above. However, there are a number of disadvantages associated with milling and micronisation processes including that the fraction having the desired particle size may be relatively small, that there may be generated a significant fraction of particles that are finer than is desired (which may be deleterious e.g. if it affects bioavailability) and that product losses generally may be considerable (e.g. through coating of the machinery). A further property of micronised products is that the surfaces of the particles generated are generally substantially amorphous (i.e. have minimal crystallinity). This may be undesirable when there exists a tendency for the amorphous regions to convert to a more stable crystalline state. Furthermore micronised or milled products may be more susceptible to moisture uptake than crystalline products. Micronisation and milling processes also suffer from the disadvantages that they are relatively energy intensive and require containment and other measures to avoid the risk of dust explosion.

WO 96/32095 describes a process for the preparation of crystalline particles for inhalation comprising mixing a solution of the active with an anti-solvent. The resulting suspension is spray-dried.

International patent application PCT/GB99/04368 (filed but not published before the priority date of this application) describes a process and apparatus for preparing particles which comprises mixing in the presence of ultrasonic radiation a flowing solution of a substance in a liquid solvent with a flowing liquid antisolvent for said substance. International patent application PCT/GB00/04237 describes a process which comprises admitting a stream of a solution of a substance in a liquid solvent and a stream of liquid antisolvent for said substance tangentially into a cylindrical mixing chamber, consequently causing a vortex which results in precipitation of crystalline particles. However, the disadvantage with these 2 processes is that particle growth or agglomeration may occur in the course of isolating the particles from the solvent/anti-solvent mixture. We have now invented an improvement to these processes which is less susceptible to the above mentioned disadvantage.

Thus, according to a first aspect of the invention there is provided a process for preparing crystalline particles of a substance which comprises mixing a flowing solution of the substance in a liquid solvent with a flowing liquid antisolvent for said substance in order to generate a suspension of crystalline particles in the solvent/anti-solvent mixture, and collecting the resultant crystalline particles generated characterised in that the solvent is more volatile than the anti-solvent and that the process further comprises the step of removing the solvent from the solvent/anti-solvent mixture prior to collection of the crystalline particles.

In a first preferred embodiment of the present invention said mixing comprises mixing in a continuous flow cell in the presence of ultrasonic radiation.

In a second preferred embodiment of the present invention said mixing comprises admitting a stream of solution of the substance in a liquid solvent and a stream of liquid antisolvent for said substance tangentially into a cylindrical mixing chamber having an axial outlet port such that said streams are thereby intimately mixed through formation of a vortex and precipitation of crystalline particles of the substance is thereby caused.

Preferably, the solvent will be miscible with the anti-solvent.

Preferably the step of removal of solvent does not give rise to removal of anti-solvent to an appreciable extent. More preferably the solvent and anti-solvent are removed in separate (e.g. sequential) steps.

Preferably, the step of removing the solvent is achieved by distillation at or below atmospheric pressure, especially vacuum distillation.

According to a further aspect of the present invention, the step of removing the solvent from the solvent/anti-solvent mixture prior to collection of the crystalline particles comprises the step of:
(a) distillation of the suspension of crystalline particles in the solvent/anti-solvent mixture at or below atmospheric pressure in order to remove the solvent;
and the step of collection of the crystalline particles comprises the steps of:
(b) cooling the resultant suspension of crystallisation particles in the anti-solvent; and
(c) collecting crystalline particles by removal of the antisolvent from the cooled suspension.

It will be appreciated that the solvent removal step refers to the removal of a significant proportion of the solvent from the solvent/antisolvent mixture. Preferably, all or substantially all solvent is removed. The benefits of the invention are expected to be greatest when solvent is removed to the greatest extent.

Preferably, in step (b) the suspension of crystalline particles obtained in step (a) will be cooled to freezing point. Also preferably, in step (b) the suspension of crystalline particles obtained in step (a) will be cooled to freezing point using a solid carbon dioxide cooling bath containing a suitable solvent eg. acetone, IMS or methanol.

Where possible, preferably the antisolvent will be water. Preferably, in step (c) the removal of the antisolvent from the cooled suspension is achieved by freeze drying.

The process of the present invention has the advantage of maintaining the original particle diameter of the particles of substance achieved by crystallisation. Conventional collection techniques involve further incubation of the particles in the solvent/antisolvent mixture which may result in undesirable effects such as crystal growth. Wherein the particles are prepared for inhalation therapy, crystal growth is disadvantageous because the particles may grow to a diameter such that they may not be effectively delivered to the lower respiratory airways.

The advantages that the invention may possess include the fact that the process may be performed in a continuous manner without requirements for batch processing, that the process may be scaled up with relative ease and that the process is capable of producing particle size distributions of very high uniformity index.

Surprisingly, the present invention provides processes for removing the solvent from the solvent/antisolvent mixture in order to prevent crystal growth, and as demonstrated in the Examples, also results in particles with more refined particle sizes than achieved with conventional harvesting techniques. Furthermore, when the antisolvent is water, once the solvent has been removed from the solvent/antisolvent mixture (by either procedure) and the mixture is cooled to freezing point, the freeze drying step ensures that the water molecules sublime from the mixture leaving only particles containing the desired substance(s).

The process of the present invention is particularly suitable for preparing particles of substances which are pharmaceutical or carrier substances suitable for inhalation therapy.

Substances suitable for inhalation therapy include substances applied topically to the lung and nose.

Examples of pharmaceutical substances suitable for inhalation therapy include analgesics, e.g., codeine, dihydromorphine, ergotamine, fentanyl or morphine; anginal preparations, e.g., diltiazem; antiallergics, e.g., cromoglycate, ketotifen or nedocromil; antiinfectives e.g., cephalosporins, penicillins, streptomycin, sulphonamides, tetracyclines and pentamidine; antihistamines, e.g., methapyrilene; anti-inflammatories, e.g., beclomethasone (eg. as the dipropionate), fluticasone (eg. as the propionate), flunisolide, budesonide, rofleponide, mometasone (e.g. as the furoate) or triamcinolone (e.g. as the acetonide); antitussives, e.g., noscapine; bronchodilators, e.g., albuterol (eg. as the sulphate), salmeterol (eg. as the xinafoate), ephedrine, adrenaline, fenoterol (eg. as the hydrobromide), formoterol (e.g. as the fumarate), isoprenaline, metaproterenol, phenylephrine, phenylpropanolamine, pirbuterol (eg. as the acetate), reproterol (eg. as the hydrochloride), rimiterol, terbutaline (eg. as the sulphate), isoetharine, tulobuterol or (-)-4-amino-3,5-dichloro-α-[[[6-[2-(2-pyridinyl)ethoxy] hexyl]methyl] benzenemethanol; diuretics, e.g., amiloride; anticholinergics, e.g., ipratropium (e.g. as the bromide), tiotropium, atropine or oxitropium; hormones, e.g., cortisone, hydrocortisone or prednisolone; xanthines, e.g., aminophylline, choline theophyllinate, lysine theophyllinate or theophylline; therapeutic proteins and peptides, e.g., insulin or glucagon; and salts, esters and solvates of any of the above. Other examples include 4-hydroxy-7-[2-[[2-[[3-(2-phenylethoxy)propyl]sulfonyl]ethyl] amino]ethyl-2(3H)-benzothiazolone or butixicort and salts and solvates thereof. Another example of a pharmaceutical substance suitable for inhalation therapy is 6α, 9α-difluoro-11β-hydroxy-16α-methyl-3-oxo-17α-propionyloxy-androsta-1,4-diene-17β-carbothioic acid S-(2-oxo-tetrahydro-furan-3-yl) ester or a solvate thereof (which compound is especially suitable for administration by the nasal route).
Other examples of pharmaceutical substances suitable for inhalation therapy which are of particular interest are:
(2R,3R,4S,5R)-2-[6-Amino-2-(1S-hydroxymethyl-2-phenyl-ethylamino)-purin-9-yl]-5-(2-ethyl-2H-tetrazol-5-yl)-tetrahydro-furan-3,4-diol or a salt thereof (eg. the maleate salt); and
(2S)-3-[4-({[4-(Aminocarbonyl)-1-piperidinyl]carbonyl}oxy)phenyl]-2-[((2S)-4-methyl-2-{[2-(2-methylphenoxy)acetyl]amino}pentanoyl)amino] propanoic acid or a salt thereof (eg. as free acid or potassium salt).

Examples of other pharmaceutical substances for which the process according to the invention is useful include compounds to be administered orally such as 2(S)-(2-benzoyl-phenylamino)-3-{4-[2-(5-methyl-2-phenyl-oxazol-4-yl)-ethoxy]-phenyl}-propionic acid, 2,6-diamino-3-(2,3,5-trichlorophenyl)pyrazine and naratriptan (eg. as hydrochloride) and other 5HT-1 agonists such as sumatriptan (eg. as succinate). Another compound of interest is (S)-[2-(1-iminoethylamino)ethyl]-L-homocysteine or a salt or racemate thereof (eg. preferably the 2- isomer).

Pharmaceutical substances as described above include asymmetric molecules which may exist as mixtures of optical isomers (e.g. as racemates) or as purified single enantiomers.

Pharmaceutical substances of particular interest include fluticasone, beclomethasone, salmeterol, salbutamol or an ester, salt or solvate thereof. The substance of most interest is salmeterol xinafoate (including the racemate or the purified r- or s- enantiomers). Fluticasone propionate is also of particular interest.
Examples of carrier substances include lactose.

The solvent and antisolvent liquids will be selected so as to be appropriate for the substance. Preferably, they are readily miscible in the proportions employed. Suitable combinations of solvent/antisolvent include acetone/water, ethanol/IPA, methanol/IPA, methanol/water and reciprocal pairs. Methanol/IPE is also a suitable pairing.

For generation of small particles by the process according to the invention, it is preferred that the difference between the dissolution properties of the solvent and anti-solvent be as great as possible. For reasons of industrial efficiency (particularly in order to reduce the throughput volumes of liquid) it is preferred to use concentrations of substance in solvent which are as high as possible. Nevertheless the solutions must be stable and not prone to crystallisation before discharge into the continuous flow cell. With this end in mind, it may be preferred to use the solution of the substance in the solvent at elevated temperature. It may also be preferable to cool the anti-solvent.

In order to prevent premature precipitation of the dissolved substance in the lines it will generally be desired to prime the apparatus by first pumping it with solvent. It may be preferred to prime the apparatus by pumping it with heated solvent, particularly when the dissolved substance is close to its solubility limit.

When the substance is fluticasone propionate we prefer the solvent to be acetone and the anti-solvent to be water.
When the substance is salmeterol xinafoate we prefer the solvent to be methanol or acetone (more preferably methanol) and the anti-solvent to be water.
When the substance is salbutamol sulphate we prefer the solvent to be water and the anti-solvent to be IMS.
When the substance is beclomethasone dipropionate we prefer the solvent to be IMS and the anti-solvent to be water.
When the substance is lactose we prefer the solvent to be water and the anti-solvent to be ethanol.
When the substance is budesonide, we prefer the solvent to be methanol and the anti-solvent to be water.
When the substance is formoterol fumarate or terbutaline sulphate we prefer the solvent to be methanol or acetone and the anti-solvent to be water.
When the substance is 2,6-diamino-3-(2,3,5-trichlorophenyl)pyrazine
we prefer the solvent to be methanol and the anti-solvent to be water.
When the substance is 2(S)-(2-benzoyl-phenylamino)-3-{4-[2-(5-methyl-2-phenyl-oxazol-4-yl)-ethoxy]-phenyl}-propionic acid we prefer the solvent to be acetone and the anti-solvent to be water.
When the substance is naratriptan hydrochloride we prefer the solvent to be methanol and the antisolvent to be IPE.
When the substance is 6α, 9α-difluoro-11β-hydroxy-16α-methyl-3-oxo-17α-propionyloxy-androsta-1,4-diene-17β-carbothioic acid S-(2-oxo-tetrahydro-furan-3-yl) ester we prefer the solvent to be acetone and the anti-solvent to be water.

We have found that the method according to the invention is suitable for producing populations of mixtures when the substance is a mixture of substances. When the substance is a mixture the method has particular advantages since it is capable of producing mixtures of crystalline particles of very high homogeneity without the need for any blending step. When the substance is a mixture the solvent and anti-solvent will have to be appropriate for all components of the mixture. Differential solubilities in the recrystalline mixture tend to result in the output proportions of the mixture differing from the initial proportions in solution in the solvent and so appropriate adjustment of the input proportions to achieve the desired output proportions may be necessary.

The method according to the invention is particularly suitable for producing mixtures of crystalline particles of salmeterol and fluticasone or salts and esters thereof e.g. salmeterol xinafoate and fluticasone propionate. The preferred solvent is acetone. The preferred anti-solvent is water. Recrystallisation from acetone using water as anti-solvent tends to cause an increase in the ratio of salmeterol xinafoate to fluticasone propionate relative to their proportion in solution in acetone. The method is also expected to be suitable for producing mixtures of crystalline particles of formoterol and budesonide or salts and esters thereof e.g. formoterol fumarate and budesonide.

As a further aspect of the invention we provide a population of particles obtainable by a process according to the invention.

Particles of pharmaceutical or carrier substances may be obtained which are suitable for use in a pharmaceutical composition for inhalation therapy, such as dry powder composition (whether containing pure drug, or drug mixed with a carrier such as lactose) or a pressurised liquid formulation (e.g. a formulation comprising a hydrofluoroalkane (HFA) propellant such as HFA134a or HFA227 or a mixture thereof).

Pressurised liquid formulations suitable for metered-dose inhalers will be retained in canisters, typically aluminium canisters (which may be plastics lined) which are provided with a metering valve of appropriate metering volume.

It will be appreciated that references to inhalation therapy also extend to administration of pharmaceutical compositions via the nasal route. Formulations suitable for nasal delivery include pressurised (e.g. HFA containing) formulations and non pressurised (e.g. aqueous) formulations which may be metered by the delivery device adapted for administration to the nose:

We also provide a pharmaceutical composition comprising a population of particles prepared according to the invention.

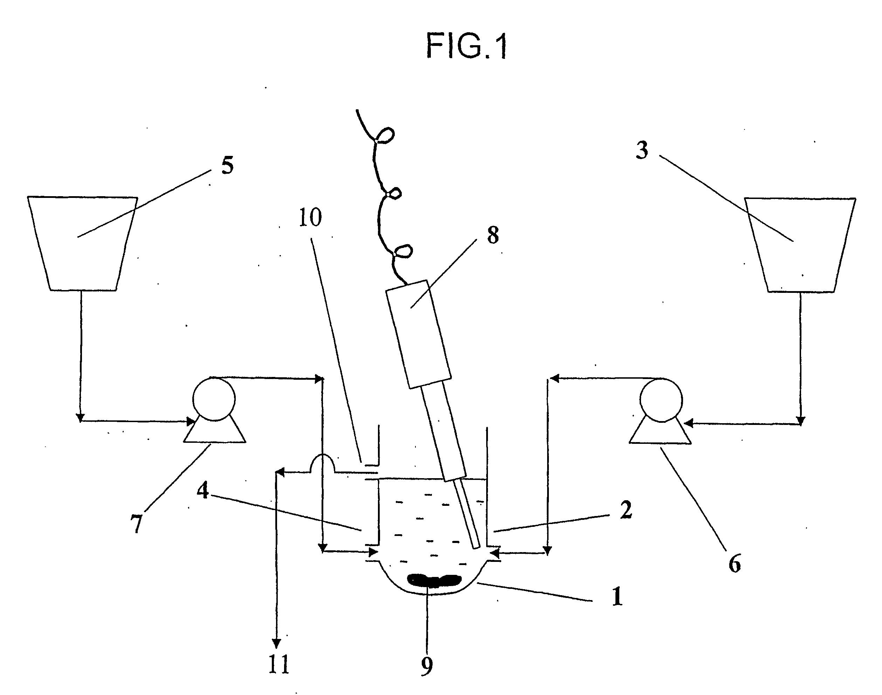

Apparatus suitable for use in the present invention is illustrated by reference to Figure 1 in which mixing chamber 1 is provided with first inlet port 2 connected to first reservoir 3 containing substance dissolved in solvent and second inlet port 4 connected to second reservoir 5 containing anti-solvent. Pumps 6 and 7 deliver liquid from reservoirs 3 and 5 to mixing chamber 1 at a controlled rate. An ultrasound probe 8 is located in the vicinity of, and just above, inlet port 2. When pumps 6 and 7 are in operation, liquids from reservoirs 3 and 5 are delivered to mixing chamber 1 and are mixed with the aid of magnetic stirrer 9. Liquid containing the particles of substance thus generated flows out of the mixing chamber via exit port 10. The solvent within this flowing suspension is then removed using vacuum distillation 11 according to the present invention.

### Brief description of the drawings.

Figure 1: Example apparatus according to the invention

The present invention is illustrated by the following example:

### Examples

### Example 1

### Distributions of particles of crystalline fluticasone propionate Experimental procedure

The drug substance (fluticasone propionate, FP) (10g) was dissolved in hot acetone (150ml) and then allowed to cool to ambient temperature (20 °C). A flow cell was then charged with a 4:1 mixture of water and acetone respectively. Pump 1 (containing the fluticasone propionate in acetone) was set at a flow rate of 20ml/min. Pump 2 (containing chilled water) was set at 80ml/min. An ultrasound probe was set to deliver approximately 70-75watts of power. When the ultrasound probe and both pumps were turned on, rapid onset of crystallisation occurred.

The slurry output was collected in a Bucci flask and concentrated *in vacuo* until all of the acetone had been removed, leaving only aqueous slurry (approximately 50ml). This was then rapidly frozen using a solid carbon dioxide cooling bath containing acetone. The contents of the flask were then freeze dried overnight to give a free flowing fine dry white powder (Example 1A). No sieving or deaggregation of the particles by passing them through a screen was necessary.

Control particles were prepared by suspending fluticasone propionate (10g) (obtained from micronisation rather than the process described for Example 1A) in water (50ml) and freezing rapidly using a solid carbon dioxide cooling bath containing acetone. The resultant mixture was then freeze dried overnight to give Example 1B.

### Analysis

Particles of Examples 1A and 1B were analysed using Malvem laser diffraction particle sizing.
- Instrument:: Malvern Mastersizer X
- Lens:: 45mm Reverse Fourier
- Analysis:: 0607 presentation code
- Dispersant:: Iso Octane / Lecithin 0.05% w/w
- Pre dispersion:: Sonicate for 3 minutes
- Obscuration:: 10% to 16%

One analysis per sample was carried out. The median particle size (D50), particle size at 90% undersize (D90) and particle size at 10% undersize (D10) were used as responses to characterise the medium, course, and fine particles.

### Results

| Example | D10 (µm) | D50 (µm) | D90 (µm) |
|---|---|---|---|
| 1A | 1.01 | 2.86 | 5.93 |
| 1B | 1.77 | 3.65 | 7.24 |

The results of this investigation indicate that the process of the present invention produces particles with more refined particle sizes than particles obtained from micronisation.

## Claims

1. A process for preparing crystalline particles of a substance which comprises mixing a flowing solution of the substance in a liquid solvent with a flowing liquid antisolvent for said substance in order to generate a suspension of crystalline particles in the solvent/anti-solvent mixture, and collecting the resultant crystalline particles generated **characterised in that** the solvent is more volatile than the anti-solvent and that the process further comprises the step of removing the solvent from the solvent/anti-solvent mixture prior to collection of the crystalline particles.

2. A process according to claim 1 wherein said mixing comprises mixing in a continuous flow cell in the presence of ultrasonic radiation.

3. A process according to claim 1 wherein said mixing comprises admitting a stream of solution of the substance in a liquid solvent and a stream of liquid antisolvent for said substance tangentially into a cylindrical mixing chamber having an axial outlet port such that said streams are thereby intimately mixed through formation of a vortex and precipitation of crystalline particles of the substance is thereby caused.

4. A process according to any one of claims 1 to 3 wherein the solvent is miscible with the anti-solvent.

5. A process according to any one of claims 1 to 4 wherein the step of removal of the solvent does not give rise to removal of the anti-solvent.

6. A process according to any one of claims 1 to 5 wherein the solvent and anti-solvent are removed in separate steps.

7. A process according to any one of claims 1 to 6 wherein the step of removing the solvent is achieved by distillation.

8. A process according to any one of claims 1 to 7 wherein the step of removing the solvent from the solvent/anti-solvent mixture prior to collection of the crystalline particles comprises the step of:
(a) distillation of the suspension of crystalline particles in the solvent/anti-solvent mixture at or below atmospheric pressure in order to remove the solvent;
and the step of collection of the crystalline particles comprises the steps of:
(b) cooling the resultant suspension of crystallisation particles in the anti-solvent; and
(c) collecting crystalline particles by removal of the antisolvent from the cooled suspension.

9. A process according to claim 7 or claim 8 wherein the step of removing the solvent is achieved by vacuum distillation.

10. A process according to any one of claims 1 to 9 wherein all or substantially all solvent is removed in the solvent removal step.

11. A process according to any one of claims 8 to 10 wherein in step (b) the suspension of crystalline particles obtained in step (a) is cooled to freezing point.

12. A process according to any one of claims 8 to 11 wherein in step (b) the suspension of crystalline particles obtained in step (a) are cooled to freezing point using a solid carbon dioxide cooling bath containing a suitable solvent eg. acetone, IMS or methanol.

13. A process according to any one of claims 1 to 12 wherein the antisolvent is water.

14. A process according to any one of claims 8 to 12 wherein in step (d) the removal of the antisolvent from the cooled suspension is achieved by freeze drying.

15. A process according to any one of claims 1 to 14 wherein the process prepares particles of substances which are pharmaceutical or carrier substances suitable for inhalation therapy.

16. A process according to claim 15 wherein the substance is fluticasone, beclomethasone, salmeterol, salbutamol or an ester, salt or solvate thereof.

17. A process according to claim 15 wherein the substance is lactose.

18. A process according to claim 15 wherein the substance is 6α, 9α-difluoro-11β-hydroxy-16α-methyl-3-oxo-17α-propionyloxy-androsta-1,4-diene-17β-carbothioic acid S-(2-oxo-tetrahydro-furan-3-yl) ester.

19. A process according to claim 16 wherein the substance is fluticasone propionate.

20. A process according to claim 16 wherein the substance is salmeterol xinafoate.

21. A process according to claim 13 wherein the substance is a mixture.

22. A process according to claim 21 wherein the substance is a mixture of fluticasone propionate and salmeterol xinafoate.

23. A process according to any one of claims 1 to 14 wherein the process prepares particles of substances which may be administered orally.

24. A process according to claim 23 wherein the substance is 2(S)-(2-benzoyl-phenylamino)-3-{4-[2-(5-methyl-2-phenyl-oxazol-4-yl)-ethoxy]-phenyl}-propionic acid or 2,6-diamino-3-(2,3,5-trichlorophenyl)pyrazine.

25. A process according to claim 23 wherein the substance is naratriptan hydrochloride.

26. A population of particles obtainable by a process according to any one of claims 1 to 25.

27. A pharmaceutical composition comprising a population of particles according to claim 26.

## Patentansprüche

1. Verfahren zur Herstellung kristalliner Partikel aus einer Substanz, das das Vermischen einer fließenden Lösung der Substanz in einem flüssigen Lösungsmittel mit einem fließenden flüssigen Antilösungsmittel für die Substanz, um eine Suspension kristalliner Partikel in der Lösungsmittel/Antilösungsmittel-Mischung zu erzeugen, und das Auffangen der resultierenden erzeugten kristallinen Partikel umfaßt, **dadurch gekennzeichnet, daß** das Lösungsmittel flüchtiger als das Antilösungsmittel ist und daß das Verfahren ferner den Schritt der Entfernung des Lösungsmittels aus der Lösungsmittel/Antilösungsmittel-Mischung vor dem Auffangen der kristallinen Partikel umfaßt.

2. Verfahren gemäß Anspruch 1, worin das Vermischen das Vermischen in einer Durchflußzelle in Gegenwart von Ultraschallschwingung umfaßt.

3. Verfahren gemäß Anspruch 1, worin das Vermischen das Zuführen eines Stroms von Lösung der Substanz in einem flüssigen Lösungsmittel und eines Stroms von flüssigem Antilösungsmittel für die Substanz tangential in eine zylindrische Mischkammer mit einer axialen Auslaßöffnung umfaßt, so daß die Ströme dadurch durch Bildung eines Wirbels innig vermischt werden und dadurch eine Ausfällung von kristallinen Partikeln der Substanz verursacht wird.

4. Verfahren gemäß einem der Ansprüche 1 bis 3, worin das Lösungsmittel mit dem Antilösungsmittel mischbar ist.

5. Verfahren gemäß einem der Ansprüche 1 bis 4, worin der Schritt der Entfernung des Lösungsmittel nicht zur Entfernung des Antilösungsmittels führt.

6. Verfahren gemäß einem der Ansprüche 1 bis 5, worin das Lösungsmittel und das Antilösungsmittel in separaten Schritten entfernt werden.

7. Verfahren gemäß einem der Ansprüche 1 bis 6, worin der Schritt der Entfernung des Lösungsmittels durch Destillation erreicht wird.

8. Verfahren gemäß einem der Ansprüche 1 bis 7, worin der Schritt der Entfernung des Lösungsmittels aus der Lösungsmittel/Antilösungsmittel-Mischung vor dem Auffangen der kristallinen Partikel den folgenden Schritt umfaßt:
(a) Destillation der Suspension von kristallinen Partikeln in der Lösungsmittel/Antilösungsmittel-Mischung bei oder unter Atmosphärendruck zur Entfernung des Lösungsmittels;
und der Schritt des Auffangens der kristallinen Partikel die folgenden Schritte umfaßt:
(b) Abkühlen der resultierenden Suspension von Kristallisationspartikeln im Antilösungsmittel; und
(c) Auffangen kristalliner Partikel durch Entfernung des Antilösungsmittels aus der abgekühlten Suspension.

9. Verfahren gemäß Anspruch 7 oder 8, worin der Schritt der Entfernung des Lösungsmittels durch Vakuumdestillation erreicht wird.

10. Verfahren gemäß einem der Ansprüche 1 bis 9, worin das gesamte oder im wesentliche gesamte Lösungsmittel im Lösungsmittelentfernungsschritt entfernt wird.

11. Verfahren gemäß einem der Ansprüche 8 bis 10, worin in Schritt (b) die in Schritt (a) erhaltene Suspension von kristallinen Partikeln auf den Gefrierpunkt abgekühlt wird.

12. Verfahren gemäß einem der Ansprüche 8 bis 11, worin in Schritt (b) die in Schritt (a) erhaltene Suspension von kristallinen Partikeln auf den Gefrierpunkt unter Verwendung eines Kühlbads mit festem Kohlendioxid, das ein geeignetes Lösungsmittel enthält, z.B. Aceton, Brennspiritus oder Methanol, abgekühlt wird.

13. Verfahren gemäß einem der Ansprüche 1 bis 12, worin das Antilösungsmittel Wasser ist.

14. Verfahren gemäß einem der Ansprüche 8 bis 12, worin in Schritt (d) die Entfernung des Antilösungsmittels aus der abgekühlten Suspension durch Gefriertrocknung erreicht wird.

15. Verfahren gemäß einem der Ansprüche 1 bis 14, worin das Verfahren Partikel von Substanzen herstellt, die pharmazeutische oder Trägersubstanzen sind, die zur Inhalationstherapie geeignet sind.

16. Verfahren gemäß Anspruch 15, worin die Substanz Fluticason, Beclomethason, Salmeterol, Salbutamol oder ein Ester, Salz oder Solvat davon ist.

17. Verfahren gemäß Anspruch 15, worin die Substanz Lactose ist.

18. Verfahren gemäß Anspruch 15, worin die Substanz 6α,9α-Difluor-11β-hydroxy-16α-methyl-3-oxo-17α-propionyloxy-androsta-1,4-dien-17β-thiocarbonsäure-S-(2-oxo-tetrahydrofuran-3-yl)ester ist.

19. Verfahren gemäß Anspruch 16, worin die Substanz Fluticasonpropionat ist.

20. Verfahren gemäß Anspruch 16, worin die Substanz Salmeterolxinafoat ist.

21. Verfahren gemäß Anspruch 13, worin die Substanz eine Mischung ist.

22. Verfahren gemäß Anspruch 21, worin die Substanz eine Mischung aus Fluticasonpropionat und Salmeterolxinafoat ist.

23. Verfahren gemäß einem der Ansprüche 1 bis 14, worin das Verfahren Partikel von Substanzen herstellt, die oral verabreicht werden können.

24. Verfahren gemäß Anspruch 23, worin die Substanz 2(S)-(2-Benzoyl-phenylamino)-3-{4-[2-(5-methyl-2-phenyloxazol-4-yl)ethoxy]phenyl}propionsäure oder 2,6-Diamino-3-(2,3,5-trichlorphenyl)pyrazin ist.

25. Verfahren gemäß Anspruch 23, worin die Substanz Naratriptanhydrochlorid ist.

26. Gruppe von Partikeln, erhältlich durch ein Verfahren gemäß einem der Ansprüche 1 bis 25.

27. Pharmazeutische Zusammensetzung, die eine Gruppe von Partikeln gemäß Anspruch 26 umfaßt.

## Revendications

1. Procédé de préparation de particules cristallines d'une substance qui comprend le mélange d'une solution fluide de la substance dans un solvant liquide avec un antisolvant liquide fluide pour ladite substance dans le but de générer une suspension de particules cristallines dans le mélange de solvant/antisolvant, et la récupération des particules cristallines résultantes générées, **caractérisé en ce que** le solvant est plus volatile que l'antisolvant et **en ce que** le procédé comprend en outre l'étape d'élimination du solvant à partir du mélange de solvant/antisolvant avant la récupération des particules cristallines.

2. Procédé selon la revendication 1, dans lequel ledit mélange comprend le mélange dans un compartiment d'écoulement continu en présence d'émission ultrasonique.

3. Procédé selon la revendication 1, dans lequel ledit mélange comprend l'admission d'un courant de solution de la substance dans un solvant liquide et d'un courant d'antisolvant liquide pour ladite substance tangentiellement à l'intérieur d'une chambre de mélange cylindrique ayant un port de sortie axial de sorte que lesdits courants sont de cette façon intimement mélangés par la formation d'un vortex et la précipitation des particules cristallines de la substance est provoquée de cette façon.

4. Procédé selon l'une quelconque des revendications 1 à 3, dans lequel le solvant est miscible à l'antisolvant.

5. Procédé selon l'une quelconque des revendications 1 à 4, dans lequel l'étape d'élimination du solvant n'entraîne pas l'élimination de l'antisolvant.

6. Procédé selon l'une quelconque des revendications 1 à 5, dans lequel le solvant et l'antisolvant sont éliminés dans des étapes séparées.

7. Procédé selon l'une quelconque des revendications 1 à 6, dans lequel l'étape d'élimination du solvant est effectuée par distillation.

8. Procédé selon l'une quelconque des revendications 1 à 7, dans lequel l'étape d'élimination du solvant à partir du mélange de solvant/antisolvant avant la récupération des particules cristallines comprend l'étape de :
(a) distillation de la suspension des particules cristallines dans le mélange de solvant/antisolvant à ou sous la pression atmosphérique dans le but d'éliminer le solvant ;
et l'étape de récupération des particules cristallines comprend les étapes de :
(b) refroidissement de la suspension résultante des particules de cristallisation dans l'antisolvant ; et
(c) récupération des particules cristallines par élimination de l'antisolvant à partir de la suspension refroidie.

9. Procédé selon la revendication 7 ou la revendication 8, dans lequel l'étape d'élimination du solvant est effectuée par distillation sous vide.

10. Procédé selon l'une quelconque des revendications 1 à 9, dans lequel l'intégralité du ou substantiellement tout le solvant est éliminé dans l'étape d'élimination du solvant.

11. Procédé selon l'une quelconque des revendications 8 à 10, dans lequel dans l'étape (b), la suspension de particules cristallines obtenue dans l'étape (a) est refroidie jusqu'au point de congélation.

12. Procédé selon l'une quelconque des revendications 8 à 11, dans lequel dans l'étape (b), la suspension de particules cristallines obtenue dans l'étape (a) est refroidie jusqu'au point de congélation en utilisant un bain de refroidissement au dioxyde de carbone solide contenant un solvant convenable, par exemple l'acétone, l'IMS ou le méthanol.

13. Procédé selon l'une quelconque des revendications 1 à 12, dans lequel l'antisolvant est l'eau.

14. Procédé selon l'une quelconque des revendications 8 à 12, dans lequel dans l'étape (d), l'élimination de l'antisolvant à partir de la suspension refroidie est effectuée par lyophilisation.

15. Procédé selon l'une quelconque des revendications 1 à 14, dans lequel le procédé prépare les particules de substances qui sont des substances pharmaceutiques ou porteuses adaptées à une thérapie par inhalation.

16. Procédé selon la revendication 15, dans lequel la substance est la fluticasone, la béclométhasone, le salmétérol, le salbutamol ou un ester, sel ou solvate de ceux-ci.

17. Procédé selon la revendication 15, dans lequel la substance est le lactose.

18. Procédé selon la revendication 15, dans lequel la substance est le S-(2-oxo-tétrahydro-furan-3-yl)ester de l'acide 6α, 9α-difluoro-11β-hydroxy-16α-méthyl-3-oxo-17α-propionyloxy-androsta-1,4-diène-17β-carbothioïque.

19. Procédé selon la revendication 16, dans lequel la substance est le propionate de fluticasone.

20. Procédé selon la revendication 16, dans lequel la substance est le xinafoate de salmétérol.

21. Procédé selon la revendication 13, dans lequel la substance est un mélange.

22. Procédé selon la revendication 21, dans lequel la substance est un mélange de propionate de fluticasone et de xinafoate de salmétérol.

23. Procédé selon l'une quelconque des revendications 1 à 14, dans lequel le procédé prépare des particules de substances qui peuvent être administrées par voie orale.

24. Procédé selon la revendication 23, dans lequel la substance est l'acide 2(S)-(2-benzoyl-phénylamino)-3-{4-[2-(5-méthyl-2-phényl-oxazol-4-yl)-éthoxy]-phényl}-propionique ou la 2,6-diamino-3-(2,3,5-trichlorophényl)pyrazine.

25. Procédé selon la revendication 23, dans lequel la substance est le chlorhydrate de naratriptan.

26. Population de particules produite par un procédé selon l'une quelconque des revendications 1 à 25.

27. Composition pharmaceutique comprenant une population de particules selon la revendication 26.
